# EUROPÄISCHE PATENTSCHRIFT

(11) **EP 4 216 879 B1**
(45) Veröffentlichungstag und Bekanntmachung des Hinweises auf die Patenterteilung: **22.04.2026**
(21) Anmeldenummer: 21782717.9
(22) Anmeldetag: 22.09.2021
(51) Int. Cl.: A61F 2/54, A61F 2/58, A61F 2/72, A61F 2/76, A61F 2/70

(54) **VERFAHREN ZUM EINRICHTEN EINER STEUERUNG UND ORTHOPÄDIETECHNISCHE EINRICHTUNG SOWIE COMPUTERPROGRAMMPRODUKT**
METHOD FOR CONFIGURING A CONTROLLER AND ORTHOPEDIC DEVICE, AND COMPUTER PROGRAM PRODUCT
PROCÉDÉ POUR CONFIGURER UN DISPOSITIF DE COMMANDE D'UN DISPOSITIF ORTHOPÉDIQUE, ET PRODUIT-PROGRAMME D'ORDINATEUR

(30) Priorität: 28.09.2020 DE 102020125255
(43) Veröffentlichungstag der Anmeldung: 02.08.2023
(73) Patentinhaber: Otto Bock Healthcare Products GmbH, 1110 Wien (AT)
(72) Erfinder: SEIFERT, Dirk, 1030 Wien (AT)
(74) Vertreter: Gramm, Lins & Partner Patent- und Rechtsanwälte PartGmbB
(86) Internationale Anmeldenummer: PCT/EP2021/076071
(87) Internationale Veröffentlichungsnummer: WO 2022/063834

(56) Entgegenhaltungen:
- DE-B3- 102017 119 490

## Beschreibung

Die Erfindung betrifft ein Verfahren zum Einrichten einer Steuerung einer orthopädietechnischen Einrichtung, die zumindest eine Datenverarbeitungseinrichtung, daran gekoppelte Sensoren und einen Aktuator aufweist, der durch die Datenverarbeitungseinrichtung aktiviert und/oder deaktiviert wird, wobei die Steuerung einen Basisfunktionsblock aufweist, in dem eine Basisfunktionalität des Aktuators festgelegt ist. Die Erfindung betrifft ebenfalls eine orthopädietechnische Einrichtung mit einer Datenverarbeitungseinrichtung, die zur Durchführung des Verfahrens eingerichtet und ausgebildet ist sowie ein Computerprogrammprodukt mit einem Programmcode.

Prothesen dienen dazu, nicht vorhandene oder nicht mehr vorhandene Gliedmaßen zu ersetzen. Prothesen sollen neben einer Annäherung an die äußere Form die zu ersetzende Gliedmaße auch in ihrer Funktion möglichst vollständig nachbilden. Neben rein mechanischen Prothesen existiert eine Vielzahl von Prothesen, die elektronisch gesteuert sind. Die Steuerung bezieht sich beispielsweise auf die Anpassung von Protheseneinrichtungen an unterschiedliche Nutzungsbedingungen oder Nutzungsanforderungen. Häufig weisen Prothesen mehr als eine Komponente auf, die zueinander verstellt oder verlagert werden können. Beispielsweise sind Prothesengelenke zur Verschwenkung eines Oberteils relativ zu einem Unterteil vorgesehen, zwischen dem Oberteil und dem Unterteil ist eine Widerstandseinrichtung oder ein Antrieb angeordnet, die oder der auf Grundlage von Sensordaten, die in einer Steuerungseinrichtung ausgewertet werden, verändert werden kann. Ein Aktuator, beispielsweise ein Motor oder eine andere Verstelleinrichtung kann Ventile verstellen, um Bewegungswiderstände zu verändern. Ein elektrischer Antrieb kann in einen Generatormodus geschaltet werden, um einen Widerstand gegen eine Verschwenkung zu bewirken, alternativ kann der Antrieb aktiviert werden, um eine Bewegung auszuführen oder zu unterstützen. Ein Magnetfeld kann erzeugt werden, beispielsweise über einen Elektromagneten, um Viskositätseigenschaften eines magnetorheologischen Mediums zu verändern. Der Elektromagnet wäre dann ein entsprechender Aktuator. Entsprechendes gilt für andere verlagerbare Einrichtungen oder Komponenten, bei denen ein Aktuator verstellt werden kann.

Orthesen sind orthopädietechnische Hilfsmittel, die an einer existierenden Gliedmaße angelegt werden und die Bewegungen führen, einschränken oder unterstützen. Zwischen gelenkig miteinander verbundenen Komponenten können Antriebe oder Widerstandseinrichtungen angeordnet sein, die korrespondierend zu Einrichtungen an Prothesen verstellt oder eingestellt werden können. Auch hier erfolgt die Verstellung auf Basis von Sensordaten, die einer Datenverarbeitungseinrichtung übermittelt werden. Die Übermittlung der Sensordaten und der Befehle zur Verstellung an den Aktuator kann drahtlos erfolgen. Unter Orthesen werden im Rahmen dieser Anmeldung auch Exoskelette verstanden, die an dem Körper eines Patienten angelegt werden und eine äußere Stützstruktur bilden, insbesondere um die Bewegungen eines Nutzers zu führen und zu beeinflussen, z.B. durch Antriebe zu unterstützen oder über Widerstandseinrichtungen zu bremsen. Orthesen und Exoskelette als Spezialfälle können neben der Unterstützung der täglichen Verrichtungen auch zu Trainingszwecken oder zu therapeutischen Zwecken verwendet und eingesetzt werden.

Aktuelle Prothesen oder Orthesen mit sensorbasierten Verstelleinrichtungen oder Aktuatoren werden mit einer Software ausgeliefert, die an den jeweiligen Patient angepasst und konfiguriert wird. Bei der Konfiguration können einzelne Parameter verändert werden, beispielsweise um das Dämpfungsverhalten einer Widerstandseinrichtung an den jeweiligen Anwender anzupassen. Ebenfalls ist es möglich, einzelne Funktionen zu aktivieren oder zu deaktivieren, beispielsweise weil der entsprechende Patient diese Funktion nicht ausführen kann oder nicht ausführen soll.

Weiterhin ist es möglich, sogenannte Zusatzmodi, die über die Grundfunktionalität hinausgehen, zu konfigurieren. Über die Zusatzmodi werden beispielsweise besondere Tätigkeiten, wie das Fahrradfahren oder Skifahren unterstützt. Die Zusatzmodi können von dem jeweiligen Anwender ausgewählt werden, eine Anpassung der jeweiligen Parameter der Zusatzmodi kann erfolgen. Eine entsprechende Auswahl erfolgt auch bei Orthesen der unteren Extremität.

Prothesen der oberen Extremität können ebenfalls individualisiert werden. Prothesenhände verfügen über bestimmte Griffmodi, die individualisiert werden können. Die Einstellung erfolgt von dem jeweiligen Orthopädietechniker oder dem Anwender, zwischen den einzelnen Griffarten kann der jeweilige Nutzer individuell umschalten.

Sowohl die Programmierung als auch die Einstellung über die Anpassung einzelner Parameter ist aufwendig. Die Möglichkeiten der Programmierung sind meistens sehr stark eingeschränkt, oder gar nicht vorhanden. Darüber hinaus besteht die Gefahr, dass eine Anpassung eines Zusatzmodus negative Auswirkungen auf bereits vorhandene Funktionalitäten hat.

Aufgabe der vorliegenden Erfindung ist es daher, ein Verfahren zum Einrichten einer Steuerung einer orthopädietechnischen Einrichtung, insbesondere einer Orthese oder Prothese oder eines Exoskelettes bereitzustellen, mit dem eine Fehlprogrammierung und Fehlkonfiguration der Steuerung vermieden werden kann. Gleichzeitig soll die Steuerung in hohen Maße anpassbar sowie die Programmierung intuitiv und flexibel sein. Es soll eine einfache und sichere Handhabung für den jeweiligen Anwender gewährleistet werden.

Die DE 10 2017 119 490 A1 betrifft ein Verfahren zur Überprüfung der Funktionsfähigkeit eines Prothesensystems mit mehreren Sensoren, zumindest einer Steuerungseinrichtung, die mit den Sensoren gekoppelt ist und Sensorsignale der Sensoren verarbeitet, zumindest einem Aktuator, der mit der Steuerungseinrichtung gekoppelt ist und der auf der Grundlage von Steuerungssignalen der Steuerungseinrichtung aktivierbar oder deaktivierbar ist, wobei zumindest eine beweglich gelagerte Prothesenkomponente über den Aktuator verlagerbar ist. In der Steuerungseinrichtung ist ein Standardprogramm hinterlegt oder von ihr abrufbar, mit dem jedem Sensor eine Aktuatoraktion zugeordnet wird und nach einer Aktivierung des Standardprogramms und dem Auslösen eines Sensorsignals eine Aktuatoraktion veranlasst und deren Ausführung oder Nichtausführung erfasst wird und/oder das Vorhandensein oder Nichtvorhandensein eines Sensorsignals auf einer Ausgabeeinrichtung ausgegeben wird.

Erfindungsgemäß wird diese Aufgabe durch ein Verfahren mit den Merkmalen des Hauptanspruches sowie eine orthopädietechnische Einrichtung und ein Computerprogrammprodukt mit den Merkmalen des jeweiligen unabhängigen Anspruchs gelöst. Vorteilhafte Ausgestaltungen und Weiterbildungen der Erfindung sind in den Unteransprüchen, der Beschreibung sowie den Figuren offenbart.

Das Verfahren zum Einrichten einer Steuerung einer orthopädietechnischen Einrichtung, insbesondere einer Orthese, einer Prothese oder eines Exoskelettes, die zumindest eine Datenverarbeitungseinrichtung, daran gekoppelte Sensoren und einen Aktuator aufweist, der durch die Datenverarbeitungseinrichtung aktiviert und/oder deaktiviert wird, wobei die Steuerung einen Basisfunktionsblock aufweist, in dem eine Basisfunktionalität des Aktuators festgelegt ist, sieht vor, dass aus einem Speicher mehrere Zusatzfunktionsblöcke, die jeweils unterschiedliche Zusatzfunktionalitäten aufweisen, einer Interfaceeinrichtung bereitgestellt werden und dass über die Interfaceeinrichtung zumindest ein Zusatzfunktionsblock ausgewählt und dem Basisfunktionsblock hinzugefügt wird, wobei eine Schnittstelle zwischen dem Basisfunktionsblock und dem zumindest einen Zusatzfunktionsblock vorhanden ist, an der die Kompatibilität der jeweiligen Funktionalitäten der Funktionsblöcke überprüft und eine Gesamtfunktionalität erzeugt wird, wobei bei anfänglich nicht vorhandener Kompatibilität eine Anpassung zumindest einer Funktionalität zur Herstellung der Kompatibilität vorgenommen oder eine Einbindung der Zusatzfunktionalität abgelehnt wird. Zum Einrichten einer Steuerung stehen somit mehrere Zusatzfunktionsblöcke bereit, die mit dem Basisfunktionsblock kombiniert werden können. Der Basisfunktionsblock umfasst die Basisfunktionalitäten des Aktuators. Bei einer Steuerung einer Prothese oder Orthese mit einem verstellbaren Widerstand kann dies die Anpassung an die jeweilige Bewegungssituation sein. Die Orthesen oder Prothesen können auch an oberen Extremitäten angeordnet werden und neben passiven Widerstandseinrichtungen wie hydraulischen Dämpfern, Bremsen und magnetorheologisch beeinflussbaren Widerstandseinrichtungen alternativ oder ergänzend aktive Antriebe wie Elektromotoren oder andere Verstelleinrichtungen aufweisen. Beim Gehen in der Ebene ist es notwendig, unterschiedliche Widerstände gegen eine Flexion oder Extension in einem künstlichen Kniegelenk bereitzustellen. Dazu werden auf Grundlage von Sensordaten, die in der Datenverarbeitungseinrichtung ausgewertet werden, entsprechende Widerstände verstellt, um ein an das Gehen mit einem natürlichen Kniegelenk angepasstes Gangverhalten zu erreichen. So kann beispielsweise zu Beginn der Standphase nach einem Fersenstoß eine Standphasenflexion zunächst ermöglicht werden, indem der Flexionswiderstand verringert wird. Im weiteren Verlauf wird der Flexionswiderstand wieder vergrößert, um eine zu große Standphasenflexion zu verhindern. Anschließend wird im Rahmen der Überrollbewegung eine Beibehaltung eines hohen Flexionswiderstandes eingestellt, um dann am Ende der Standphase eine Flexion zu ermöglichen, damit das Prothesenkniegelenk oder Orthesenkniegelenk einbeugt und ein Durchschwingen beim Vorwärtsbewegen zu ermöglichen. Diese Basisfunktionalität für das Gehen in der Ebene kann durch eine Zusatzfunktion ergänzt werden, die in einem Steuerungsblock als Zusatzfunktionsblock abgelegt ist. Eine solche Zusatzfunktion kann beispielsweise das Gehen auf einer Rampe, das Treppaufsteigen oder das Gehen mit einer erhöhten Gehgeschwindigkeit sein. Dabei kann es sein, dass der jeweils ausgewählte Zusatzfunktionsblock mit Teilen des Basisfunktionsblockes nicht kompatibel ist oder auch mit einem bereits vorhandenen Zusatzfunktionsblock kompatibel ist. Ebenso können Kompatibilitäten aus bereits vorhandenen Kombinationen aus dem Basisfunktionsblock mit einem oder mehreren Zusatzfunktionsblöcken ergeben, sodass sich bei einer bloßen Hinzufügung des Zusatzfunktionsblockes eine Gesamtfunktionalität erzeugt werden würde, die nicht vorteilhaft ist oder sogar schädlich für den Nutzer sein kann. Daher wird an der Schnittstelle zwischen dem Basisfunktionsblock und dem Zusatzfunktionsblock oder an der Schnittstelle zwischen dem Zusatzfunktionsblock an einem anderen Zusatzfunktionsblock, der bereits mit dem Basisfunktionsblock verbunden ist, eine Kompatibilitätsüberprüfung durchgeführt. Dabei werden die Funktionalitäten der einzelnen Funktionsblöcke überprüft und daraus die Gesamtfunktionalität der Steuerung erzeugt. Sofern die Kompatibilität der Funktionsblöcke nicht vorhanden ist, kann eine Anpassung zumindest einer Funktionalität, insbesondere der Funktionalität des neu hinzugefügten Zusatzfunktionsblockes erfolgen, damit die Kompatibilität erreicht wird. Auch können andere Funktionen als der des neu hinzugefügten Zusatzfunktionsblockes verändert werden. Wird beispielsweise der Funktion des neu hinzugefügten Zusatzfunktionsblockes eine größere Priorität oder Wichtigkeit eingeräumt als einer Funktionalität eines bereits hinzugefügten Zusatzfunktionsblockes, kann über die Priorisierung eine Auswahl der vorzunehmenden Anpassung erreicht werden. Alternativ zu einer Änderung einer oder mehrerer Funktionalitäten des Basisfunktionsblockes und/oder eines oder mehrerer Zusatzfunktionsblöcke ist es möglich, dass die Einbindung der Zusatzfunktionalität des hinzugefügten Zusatzfunktionsblockes abgelehnt wird, weil in dieser Zusatzfunktionalität oder in der Kompensation der neuen Zusatzfunktionalität eine Gefährdung oder eine nicht gewünschte Gesamtfunktionalität erreicht wird. Dadurch wird erreicht, dass eine Vielzahl von Zusatzfunktionalitäten in Zusatzfunktionsblöcken bereitgestellt werden kann, die ohne weitere Überprüfung miteinander kombiniert werden können, wobei automatisch eine Überprüfung der Kompatibilität und gegebenenfalls eine Anpassung der Funktionen zur Herstellung einer Kompatibilität und einer Gesamtfunktionalität durchgeführt wird. Sofern eine Anpassung nicht erreicht werden kann oder nicht erreicht werden soll, kann auch die Einbindung der Zusatzfunktionalität und damit die Kombination eines Zusatzfunktionsblockes mit dem Basisfunktionsblock oder eine Kombination eines Basisfunktionsblockes mit einem oder mehreren Zusatzfunktionsblöcken abgelehnt werden. Eine Fehlprogrammierung durch eine unzulässige Kombination von Funktionalitäten kann durch das erfindungsgemäße Verfahren nicht mehr stattfinden.

Eine Weiterbildung der Erfindung sieht vor, dass mehrere Funktionsblöcke nacheinander dem Zusatzfunktionsblock hinzugefügt werden, wobei bei jedem neuen Zusatzfunktionsblock eine Überprüfung der Kompatibilität mit der gegenwärtig vorhandenen Gesamtfunktionalität erfolgt. Es erfolgt somit ein modularer Aufbau ausgehend von dem Basisfunktionsblock, wobei in der Reihenfolge der Hinzufügung weiterer Zusatzfunktionsblöcke ausgehend von der einmal festgestellten Gesamtfunktionalität, eine weitere Hinzufügung einer Funktionalität überprüft wird. Ist nach der Hinzufügung zweier Zusatzfunktionsblöcke eine Gesamtfunktionalität erreicht, bei der einige Funktionen, die ursprünglich in einem der Zusatzfunktionsblöcke vorhanden ist, reduziert worden, wird diese bereits ausgeschlossene Funktionalität oder eine Einschränkung in dem Funktionsbereich nicht mehr berücksichtigt, da diese bereits in der vorhergehenden Gestaltungsphase ausgeschlossen wurde oder limitiert wurde. Dies hat den Effekt, dass nicht mehr alle Kombinationen aller Funktionalitäten auf die Kompatibilität zur Ausbildung einer Gesamtfunktionalität überprüft werden müssen. Vielmehr wird nur noch das neu hinzugekommene Potential an Funktionalitäten durch den neuen Zusatzfunktionsblock auf die Kompatibilität mit der bereits vorhandenen Gesamtfunktionalität hin überprüft.

Eine Weiterbildung der Erfindung sieht vor, dass über die Interface-Einrichtung zumindest ein Parameter der Basisfunktionalität und/oder der Zusatzfunktionalität verstellbar ist. Jede Funktionalität kann somit skalierbar sein, beispielsweise können Dämpfungseinstellungen zur Anpassung an individuelle Wünsche oder Gegebenheiten angepasst werden. Anpassungen können beispielsweise an Gehgeschwindigkeiten, Einsatzzwecke, Körpergewichte, Aktivitätsgrade oder auch physiologische Gegebenheiten angepasst werden. Neben der Anpassung der Parameter der Basisfunktionalität kann auch zumindest ein Parameter einer Zusatzfunktionalität erstellt werden. Der Grad der Verstellbarkeit durch die Interface-Einrichtung kann in Abhängigkeit von der erzielten Gesamtfunktionalität verändert werden. Beispielsweise kann nach Hinzufügung einer weiteren Zusatzfunktionalität ein Parameterbereich zur Verstellung einer Stellgröße der Basisfunktionalität eingeschränkt werden oder aber ein Parameterbereich der neu hinzugefügten Zusatzfunktionalität ausgenommen oder eröffnet werden, wenn eine bestimmte Gesamtfunktionalität vorhanden ist.

Jeder Zusatzfunktionalität kann ein Priorisierungswert zugeordnet werden, wobei eine Überprüfung der Kompatibilität und Anpassung der jeweiligen Funktionalität anhand der Priorisierungswerte vorgenommen wird. Weist beispielsweise eine Zusatzfunktionalität, die später hinzugefügt wird, eine höhere Priorisierung als eine bereits vorher hinzugefügte Zusatzfunktionalität auf, kann eine Umorganisierung und eine Neuausrichtung und Neuerrechnung der Gesamtfunktionalität erfolgen, die sich dann anhand der jeweiligen Priorisierungswerte ausrichtet. Somit kann unabhängig von dem Zeitpunkt oder dem Zustand der Kombination von Basisfunktionsblock und mehreren Zusatzfunktionsblöcken eine Gewichtung durchgeführt werden, anhand der ein Aufbau einer Gesamtfunktionalität durchgeführt wird. Wird ein Zusatzfunktionsblock mit der höchsten Priorität erst zu einem Zeitpunkt hinzugefügt, bei der bereits drei Zusatzfunktionsblöcke mit niedrigeren Priorisierungswerten vorhanden sind, wird die Gesamtfunktionalität neu errechnet auf der Grundlage der vorhandenen Priorisierungswerte. Bei gleichen Priorisierungswerten ist es vorteilhaft, den Zeitpunkt der Kombination oder dem Hinzufügen als Maßstab für die Reihenfolge der Überprüfung der Kompatibilitäten und der Anpassungen an Parameterwerte zu verwenden.

Vorteilhafterweise wird als Interface-Einrichtung eine grafische Benutzeroberfläche bereitgestellt, auf der die Zusatzfunktionalitäten als Benutzeroberflächenobjekte dargestellt sind. Die Darstellung als grafische Blöcke können somit durch Drag-and-Drop aus einem Menü ausgewählt und in einen Arbeitsbereich hineingezogen werden. Die Funktionsgruppen oder Zusatzfunktionsblöcke können dann über Schnittstellen miteinander verbunden werden, wobei grafische Anzeigen der jeweiligen Benutzeroberflächenobjekte andeuten können, ob eine Kombinierbarkeit überhaupt gegeben ist.

Eine Weiterbildung der Erfindung sieht vor, dass die Kompatibilität verschiedener Zusatzfunktionsblöcke miteinander oder mit der aktuellen Kombination aus Basisfunktionsblock und zumindest einem Zusatzfunktionsblock grafisch angezeigt wird. Die grafische Anzeige kann beispielsweise über Geometrien oder eine farbliche Codierung erfolgen. An den Benutzeroberflächenobjekten können eine farbliche und/oder grafische Kombinierbarkeit eines Zusatzfunktionsblockes mit dem Basisfunktionsblock und/oder einem anderen Zusatzfunktionsblock dargestellt werden. Die grundsätzliche Kombinierbarkeit kann anhand der grafischen Form der Benutzeroberflächenobjekte oder der Farbgestaltung angezeigt werden. Es können beispielsweise gleiche Farbgebungen eine grundsätzliche Kombinierbarkeit anzeigen. Ebenso kann eine Geeignetheit einer Kombinierbarkeit durch eine angenäherte Farbgebung angezeigt werden. Wird beispielsweise die Gesamtfunktionalität mit einem roten Farbton angezeigt, kann eine grundsätzliche Kombinierbarkeit mit einem roten oder orangenen Farbton angezeigt werden, eine Nichtkombinierbarkeit durch beispielsweise einen blauen oder einen grünen Farbton eines anderen Zusatzfunktionsblockes. Alternativ kann oder ergänzend kann durch eine Formänderung der Benutzeroberflächenobjekte angezeigt werden, ob eine grundsätzliche Kombinierbarkeit gegeben ist. Ist beispielsweise eine Ausnehmung in einer Kontur vorhanden, ist eine Kombinierbarkeit nur mit einer korrespondierenden Auswölbung oder einem korrespondierenden Vorsprung in einer grafischen Darstellung eines Zusatzfunktionsblockes oder der Darstellung der Gesamtfunktionalität gegeben. Die grafische Ausgestaltung oder die Formgebung der Benutzeroberflächenobjekte können sich ändern, insbesondere wenn sie Teil einer Gesamtfunktionalität geworden sind.

Vorteilhafterweise ist das Verfahren auf der Datenverarbeitungseinrichtung innerhalb der orthopädietechnischen Einrichtung implementiert. Auch die Interface-Einrichtung kann Teil der orthopädietechnischen Einrichtung sein. Eine Erleichterung der Anpassbarkeit und Bedienbarkeit ist gegeben, wenn die Interface-Einrichtung als mobiles Endgerät ausgebildet ist, beispielsweise als Mobiltelefon, Tablet, Augmented Reality Device, Virtual Reality Device, Mixed Reality Device, Head Mounted Display, wie HoloLens oder Google Glass^{®}, Sprachinterface oder auch als mobiler Computer, die oder der eine drahtlose Verbindung zu der Steuerungseinrichtung bzw. der Datenverarbeitungseinrichtung an der orthopädietechnischen Einrichtung aufweist. Grundsätzlich kann die Interfaceeinrichtung ein fester Bestandteil der orthopädietechnischen Einrichtung sein oder als separate Komponente ausgebildet und mit der übrigen orthopädietechnischen Einrichtung drahtlos oder drahtgebunden gekoppelt sein

An einem Zusatzfunktionsblock kann nicht nur eine Schnittstelle zu dem Basisfunktionsblock ausgebildet sein, vielmehr kann an einem Zusatzfunktionsblock ebenfalls eine Schnittstelle für zumindest einen weiteren Zusatzfunktionsblock ausgebildet sein, damit nach dem Hinzufügen des Zusatzfunktionsblockes weitere Zusatzfunktionsblöcke daran festgelegt und damit kombiniert werden können.

Der Basisfunktionsblock und/oder ein Zusatzfunktionsblock können mehrere Schnittstellen aufweisen, von denen zumindest eine durch eine Belegung einer anderen Schnittstelle mit einem Zusatzfunktionsblock blockiert wird. Sofern eine vorbestimmte Schnittstelle mit einem damit kompatiblen Zusatzfunktionsblock belegt wird, kann durch diese Belegung eine an sich vorhandene Schnittstelle blockiert werden und für weitere Zusatzfunktionsblöcke nicht mehr zur Verfügung stehen. Damit wird eine Ausweitung der Zusatzfunktionalität in diesem Bereich oder in dem Bereich der dort angesiedelten Funktionalität ausgeschlossen.

Eine Weiterbildung der Erfindung sieht vor, dass die Überprüfung der Kompatibilität der Funktionsblöcke und/oder die Integration mehrerer Funktionsblöcke in eine Gesamtfunktionalität in einem externen Gerät erfolgen. Das externe Gerät ist beispielsweise Teil der Interface-Einheit oder aber steht in Datenübertragungsverbindung zu der Interface-Einheit. Insbesondere über eine Funkverbindung oder über ein Datenübertragungsnetzwerk ist es möglich, Steuerungsdaten und Funktionalitäten von dem externen Gerät über die Interface-Einheit auf die orthopädietechnische Einrichtung zu übertragen. Die Interface-Einheit stellt somit eine Verbindung zwischen dem externen Gerät und der orthopädietechnischen Einrichtung bereit, wobei die Interface-Einheit entweder fest an der orthopädietechnischen Eirichtung angeordnet oder als separates, mit der übrigen orthopädietechnischen Einrichtung gekoppeltes Teil ausgebildet ist. Dadurch ist es möglich, einen gegebenenfalls notwendigen hohen Rechenaufwand zur Überprüfung und Anpassung der jeweiligen Funktionalitäten auf das externe Gerät zu verlagern und nur noch einen Anpassungsdienst oder einen Änderungsdatensatz an die Datenverarbeitungseinrichtung der orthopädietechnischen Einrichtung zu übermitteln. Durch diesen Datensatz wird dann eine jeweilige Anpassung der Gesamtfunktionalität durchgeführt und der Aktuator mit den jeweiligen Steuersignalen in Abhängigkeit von den Sensorwerten oder anderen gesteuert.

Es können vordefinierte Programme oder Vorlagen zur Erstellung eines Funktionsblockes verwendet werden, diese sogenannten Presets dienen als Basis für die Erstellung individueller Programme und können in der Datenverarbeitungseinrichtung, in der Interface-Einrichtung oder in einem externen Gerät abgelegt sein.

Die Datenverarbeitungseinrichtung weist insbesondere eine Kommunikationsschnittstelle auf, über die Daten an externe Einrichtungen gesendet oder von externen und/oder von externen Einrichtungen empfängt. Dadurch ist es möglich, ein individuell erstelltes Programm, das extern errechnet wurde, zu speichern oder aber auch ein in der orthopädietechnischen Einrichtung erstelltes Programm auf einem externen Gerät zu sichern, beispielsweise auf einem Mobiltelefon oder einem Tablet, oder über ein Netzwerk oder eine Cloud anderen mitzuteilen. Dadurch kann eine externe Wartung oder eine externe Kontrolle einer erstellten Gesamtfunktionalität durchgeführt werden. Es können durch einen Nutzer individuell erstellte Kombinationen mit unterschiedlichen Funktionalitäten und eingestellten Parametern auf Angemessenheit, Anpassbarkeit und medizinischen Nutzen überprüft werden, wenn diese Daten, gegebenenfalls in Verbindung mit Bewegungsdaten aus der orthopädietechnischen Einrichtung, an einen Orthopädietechniker oder eine andere Auswerteinstitution übermittelt werden. Insbesondere ist die Möglichkeit gegeben, eine Konfiguration eines Gelenks und/oder eine Konfiguration von Funktionsblöcken mit Hilfe der externen Einrichtung auf ein anderes Gelenk oder eine anderer orthopädietechnische Einrichtung zu transferieren.

Die orthopädietechnische Einrichtung sieht eine Datenverarbeitungseinrichtung vor, die zur Durchführung eines oben beschriebenen Verfahrens eingerichtet und ausgebildet ist, wobei eine Benutzeroberfläche vorhanden ist, die Eingabemittel zur Entgegennahme von Benutzereingaben aufweist. Die Benutzeroberfläche kann als grafische Benutzeroberfläche ausgebildet und eingerichtet sein, alternativ können andere Benutzeroberflächen vorhanden sein, beispielsweise akustische Benutzeroberflächen mit einer Sprachsteuerung oder eine Tastatur.

Das Computerprogramm mit einem Programmcode der, wenn er in einer Datenverarbeitungseinrichtung geladen ist, bringt das Verfahren, wie es oben beschrieben worden ist, zur Ausführung und ist ebenfalls Teil der Erfindung.

Nachfolgend werden Ausführungsbeispiele der Erfindung anhand der beigefügten Figuren näher erläutert. Es zeigen:
- Figur 1 -: eine schematische Darstellung einer Prothese einer oberen Extremität als orthopädietechnische Einrichtung;
- Figur 2 -: eine schematische Darstellung einer Prothese einer unteren Extremität als orthopädietechnische Einrichtung
- Figur 3 -: eine schematische Darstellung des modularen Prinzips;
- Figur 4 -: eine Variante mit Erweiterungsmöglichkeiten;
- Figur 5 -: eine Variante mit zusätzlichen Schnittstellen;
- Figur 6 -: eine Variante mit sich ausschließenden Zusatzfunktionsblöcken;
- Figur 7 -: eine Variante der Figur 6;
- Figur 8 -: eine Variante mit einstellbaren Parametern;
- Figur 9 -: ein weiteres Ausführungsbeispiel;
- Figur 10 -: eine orthopädietechnische Einrichtung in Gestalt einer Orthese;
- Figur 11 -: eine Orthese nach Figur 10 beim Gehen;
- Figur 12 -: eine Detailansicht eines externen Gerätes mit Interface-Einrichtung; sowie
- Figur 13 -: eine schematische Darstellung einer Vernetzung mehrerer orthopädietechnischer Einrichtungen.

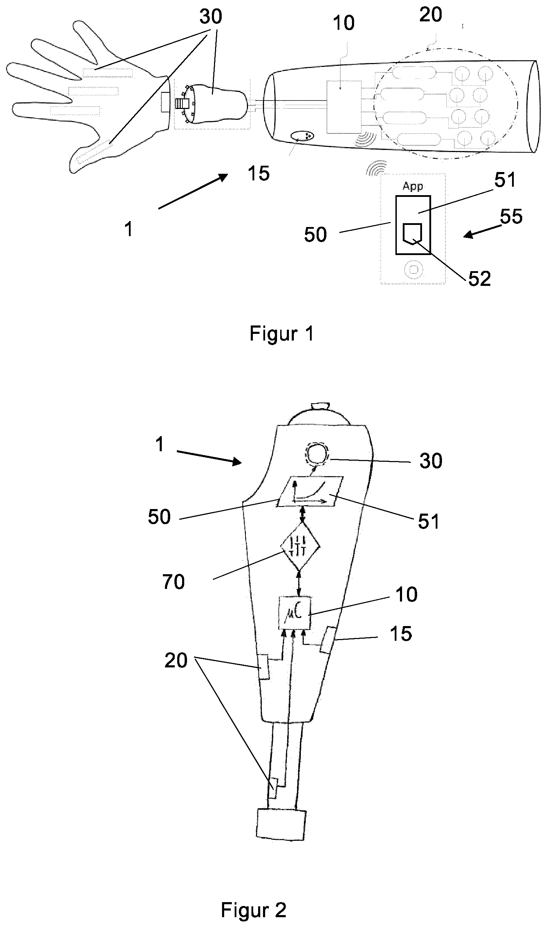

In der Figur 1 ist in einer schematischen Darstellung eine orthopädietechnische Einrichtung 1 in Gestalt einer Prothese einer oberen Extremität dargestellt. Die orthopädietechnische Einrichtung 1 weist einen Prothesenschaft zur Anlage an einem Unterarm auf. Innerhalb des Prothesenschaftes sind mehrere Sensoren 20 in Gestalt von Oberflächenelektrodenpaaren angeordnet. Diese Sensoren 20 sind mit einer Datenverarbeitungseinrichtung 10 gekoppelt, die ebenfalls innerhalb des Unterarmschaftes angeordnet sind. In der Datenverarbeitungseinrichtung 10 sind die notwendigen Softwarekomponenten und Hardwarekomponenten vorhanden, insbesondere Prozessoren, Speicher, Energiespeicher sind Kommunikationsschnittstellen, Filter und gegebenenfalls Verstärker, um die Sensordaten zu verarbeiten und aus diesen Steuerungssignale an einen Aktuator 30 zu übermitteln. In dem dargestellten Ausführungsbeispiel ist eine Vielzahl von Aktuatoren 30 in der Prothesenhand und in der Ankopplung der Prothesenhand an dem Unterarmschaft befestigt. Die Aktuatoren sind im dargestellten Ausführungsbeispiel als Elektromotoren ausgebildet, die eine Verlagerung beispielsweise der Prothesenhand um eine Drehachse in Längserstreckung des Unterarmschaftes sowie eine Verlagerung der Prothesenfinger relativ zu einem Grundkörper der Prothesenhand ermöglichen. Das Ausführungsbeispiel verdeutlicht, dass eine Vielzahl von Funktionen innerhalb der Prothesenhand verwirklicht werden kann. Unterschiedliche Griffarten, unterschiedliche Bewegungsmodi, unterschiedliche Verstellgeschwindigkeiten sowie Griffkräfte können situationsbedingt und in Abhängigkeit von dem jeweiligen Nutzer eingestellt und individualisiert werden. Zur besseren Anschaulichkeit ist ein Speicher 15 als externe Komponente dargestellt, ein solcher Speicher 15 kann selbstverständlich auch als Teil der Datenverarbeitungseinrichtung 10 ausgebildet sein.

Weiterhin ist eine drahtlose Schnittstelle zu einer Interface-Einrichtung 50 vorhanden, über die in drahtloser Art und Weise Daten ausgetauscht werden können. Die Interface-Einrichtung 50 kann als Mobiltelefon, tragbarer Computer, Tablet oder als eine andere Datenverarbeitungseinrichtung mit Benutzerschnittstelle ausgebildet sein. Die Interface-Einrichtung weist eine Benutzeroberfläche 51 auf, die beispielsweise als berührungsempfindliche Oberfläche ausgebildet sein kann. Alternativ kann die Benutzeroberfläche als Tastatur ausgebildet sein, die mit alphanumerischen Zeichen oder Funktionstasten ausgestattet ist. Die Benutzeroberfläche 51 als Tastatur kann auch elektronisch erzeugt werden. Im dargestellten Ausführungsbeispiel ist die Interface-Einrichtung 50 als ein externes Gerät 55 ausgebildet, grundsätzlich ist es auch möglich, dass die Interface-Einrichtung 50 integriert in die orthopädietechnische Einrichtung 1 ausgebildet ist, beispielsweise als Teil der Oberfläche eines Prothesenschaftes. Auf der Benutzeroberfläche 51 ist es möglich, Benutzeroberflächenobjekte 52 darzustellen, deren Funktion später näher erläutert werden wird.

Eine Variante einer orthopädietechnischen Einrichtung in Gestalt eines Prothesenkniegelenkes mit einem Oberteil und einem schwenkbar daran gelagerten Unterteil ist in der Figur 2 dargestellt. An dem distalen Ende wird ein Prothesenfuß und an dem proximalen Ende ein Oberschenkelschaft oder ein Oberschenkelrohr befestigt. Das Prothesenkniegelenk 1 weist ebenfalls eine Steuerungseinrichtung 10 mit einem Speicher 15 auf, Sensoren 20, beispielsweise Beschleunigungssensoren, Raumlagesensoren, IMU, Kraftsensoren, Temperatursensoren und dergleichen Sensoren 20 mehr sind mit der Datenverarbeitungseinrichtung 10 verbunden. An der Außenseite des Gehäuses der orthopädietechnischen Einrichtung ist die Interface-Einrichtung 50 mit der Benutzeroberfläche 51 dargestellt. Unterhalb der Interface-Einrichtung 50 sind Verstelleinrichtungen wie Schieber oder grafische Verstellsymbole dargestellt, die über eine mechanische Verstellung oder über berührungsempfindliche Oberflächen eine Veränderung von Parametern ermöglichen. Um eine Schwenkachse herum, um die das Oberteil des Prothesenkniegelenkes relativ zu dem Unterteil verschwenkbar ist, ist ein Aktuator 30 in Gestalt einer Widerstandseinrichtung angeordnet, mit dem es möglich ist, den Flexionswiderstand und/oder Extensionswiderstand in Abhängigkeit von den verarbeiteten Sensorsignalen zu verstellen. Alternative Aktuatoren 30 können vorhanden sein, beispielsweise Verstelleinrichtungen für Dämpfer, Magnetfelder für magnetorheologische Widerstandseinrichtungen, Antriebe, die als Widerstandseinrichtungen geschaltet werden können, aktive Antriebe, Energiespeicher, schaltbare Energiespeicher und dergleichen mehr.

Zur Steuerung der jeweiligen orthopädietechnischen Einrichtung werden Sensordaten von den Sensoren 20 oder Elektroden aufgenommen, in der Datenverarbeitungseinrichtung 10 verarbeitet und an den jeweiligen Aktuator 30 weitergegeben. Als Grundausstattung ist in der Datenverarbeitungseinrichtung 10 eine Basisfunktionalität eingerichtet, über die die Funktionen des jeweiligen Aktuators 30 festgelegt werden. Bei dieser Grundeinstellung oder Basisfunktionalität ist es möglich, die jeweilige orthopädietechnische Einrichtung sicher zu betreiben. Zur Anpassung an den jeweiligen Nutzer ist es wünschenswert und häufig notwendig, weitere Funktionalitäten zu implementieren, die über die Basisfunktionalität hinausgehen. Diese Zusatzfunktionalitäten sind erfindungsgemäß in sogenannten Zusatzfunktionsblöcken abgelegt, die in der jeweiligen Interface-Einrichtung 50 für den Nutzer bereitgestellt werden. Der Nutzer kann dabei entweder der Orthopädietechniker oder die Person sein, die die orthopädietechnische Einrichtung an den jeweiligen Nutzer anpasst, grundsätzlich besteht auch die Möglichkeit, dass die Anpassung durch den Nutzer der jeweiligen Prothese, Orthese oder Exoskelett oder einer anderen orthopädietechnischen Einrichtung erfolgt. In den Zusatzfunktionsblöcken oder in dem zumindest einem Zusatzfunktionsblock sind Funktionalitäten hinterlegt, die in dem Basisfunktionsblock nicht oder in einer anderen Ausgestaltung oder in einer anderen Kombination vorhanden sind. Da nicht alle Zusatzfunktionalitäten mit der Basisfunktionalität uneingeschränkt kompatibel sind, ist erfindungsgemäß vorgesehen, dass bei Hinzufügung eines Zusatzfunktionsblockes an den Basisfunktionsblock eine Überprüfung der Gesamtfunktionalität durchgeführt wird. Im Rahmen der festgestellten Kompatibilität der Kombination der Funktionsblöcke, also des Basisfunktionsblockes und des oder der Zusatzfunktionsblöcke, wird eine Gesamtfunktionalität für die orthopädietechnische Einrichtung 1 erzeugt. Ist eine vollständige Kompatibilität der Funktionalitäten von Basisfunktionsblock und Zusatzfunktionsblock anfänglich nicht vorhanden, wird eine Anpassung zumindest einer Funktionalität vorgenommen, damit eine Gesamtfunktionalität für die orthopädietechnische Einrichtung nach Hinzufügung des Zusatzfunktionsblockes erreicht werden kann. Dabei kann die Anpassung entweder in der Zusatzfunktionalität, in der Basisfunktionalität oder in beiden Funktionalitäten erfolgen, sodass insgesamt eine sichere Gesamtfunktionalität vorliegt. Grundsätzlich besteht auch die Möglichkeit, dass statt einer Anpassung des Funktionsumfanges einer oder mehrerer Zusatzfunktionalitäten oder der Basisfunktionalität eine Einbindung der ausgewählten Zusatzfunktionalität abgelehnt wird, weil diese beispielsweise nicht mit anderen Funktionalitäten, die eine höhere Priorisierung haben, kompatibel ist und die Sicherheit oder Funktionsfähigkeit des Gesamtsystems kompromittieren würde.

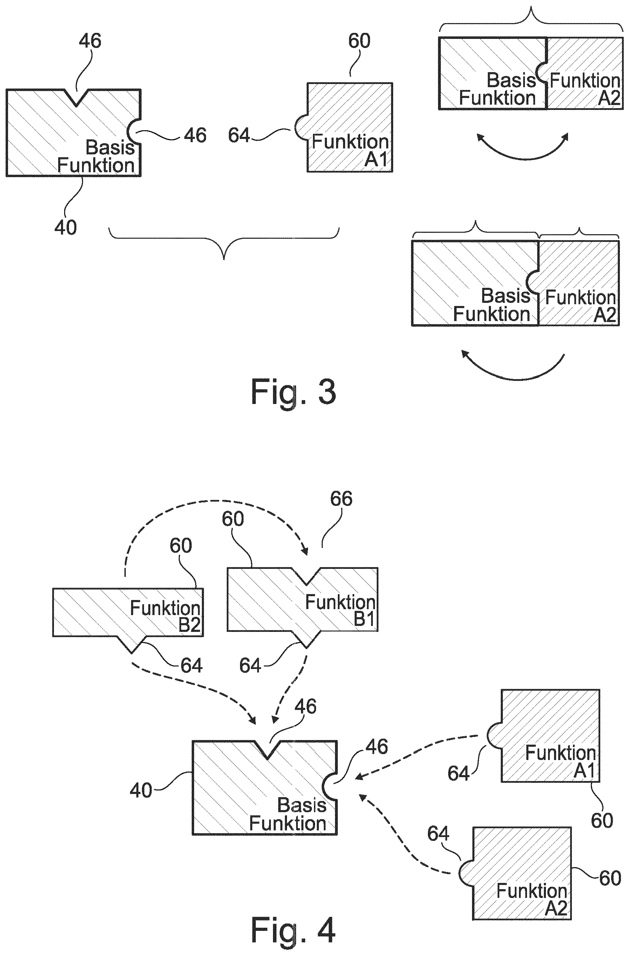

In der Figur 3 ist eine schematische Darstellung dieses modularen Prinzips gezeigt. Eine Basisfunktionalität 40 weist zwei Schnittstellen 46 auf, die grafisch unterschiedlich dargestellt sind. Ein Zusatzfunktionsblock 60 weist ebenfalls eine Schnittstelle 64 auf, die in dem dargestellten Ausführungsbeispiel nur mit einer der beiden Schnittstellen 46 des Basisfunktionsblockes 40 kompatibel ist. Wie durch die Klammer angedeutet, werden die beiden Funktionsblöcke 40, 60 miteinander verbunden. Als Ergebnis kann dabei entweder eine wechselseitige Beeinflussung der beiden Funktionsblöcke 40, 60 erfolgen, wie in der oberen rechten Darstellung der Figur 3 gezeigt ist. Alternativ ist es möglich, dass nur eine einseitige Beeinflussung von einem Funktionsblock auf den anderen erfolgt, in dem dargestellten Ausführungsbeispiel von dem Zusatzfunktionsblock 60 auf den Basisfunktionsblock 40. Die Gesamtfunktionalität ergibt sich dann je nach Abstimmung der Funktionalitäten zwischen den Funktionsblöcken 40, 60.

Eine Variante der Erfindung ist in der Figur 4 gezeigt, bei der ein Basisfunktionsblock 40, wie in der Figur 3 zwei unterschiedliche Schnittstellen 46 aufweist. Insgesamt sind vier weitere Zusatzfunktionsblöcke 60 vorgesehen, die mit dem Basisfunktionsblock 40 koppelbar sind. An der Oberseite des Basisfunktionsblocks 40 kann ein erster Zusatzfunktionsblock 60 ohne weitere Möglichkeiten einer Kombination oder ein zweiter Zusatzfunktionsblock 60 mit einer weiteren Schnittstelle 66 angekoppelt werden. Die jeweilige Schnittstelle 64, die kompatibel zu der jeweiligen Schnittstelle 46 an dem Basisfunktionsblock 40 ist, ist in dem dargestellten Beispiel auch für die Entkopplung des ersten Zusatzfunktionsblockes 60 benutzbar. Dadurch ergibt sich eine Erweiterung um zwei Zusatzfunktionsblöcke 60 an der Oberseite und der ersten Schnittstelle 46 des Basisfunktionsblockes. Die zweite Schnittstelle 46 ist mit einem anderen Zusatzfunktionsblock 60 koppelbar, diese Zusatzfunktionsblöcke 60 weisen keine weiteren Schnittstellen auf, um miteinander gekoppelt zu werden, sodass an dieser Schnittstelle 46 nur ein einzelner Zusatzfunktionsblock vorhanden ist. In dem dargestellten Ausführungsbeispiel ist somit ein Zusatzfunktionsblock 60 vorhanden, der zwei Schnittstellen 64, 46 aufweist, während die anderen Zusatzfunktionsblöcke 60 nur eine Schnittstelle 46 aufweisen.

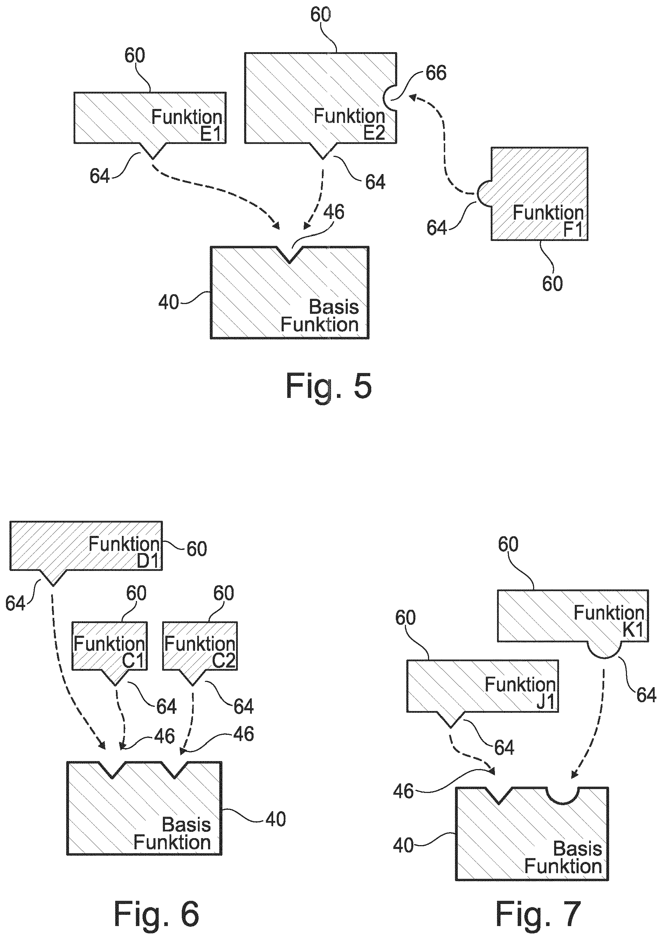

Eine Variante der Erfindung ist in der Figur 5 gezeigt, bei der ein Basisfunktionsblock 40 nur eine Schnittstelle 46 aufweist. Diese kann von zwei Zusatzfunktionsblöcken 60 besetzt werden, wobei ein Zusatzfunktionsblock 60 eine weitere Schnittstelle 66 aufweist, die mit einer Schnittstelle 64 eines weiteren Zusatzfunktionsblockes 60 gekoppelt werden kann. Somit ergibt sich für den Aufbau einer Gesamtfunktionalität die Auswahl zwischen drei Kombinationen des Basisfunktionsblockes 40 mit entweder einem ersten Zusatzfunktionsblock 60 ohne Erweiterungsmöglichkeit, einem zweiten Zusatzfunktionsblock 60 mit einer erweiterten Möglichkeit, ohne dass diese wahrgenommen worden ist und schließlich mit der Kombination des Basisfunktionsblockes 40 mit dem um einen Zusatzfunktionsblock 60 erweiterten Zusatzfunktionsblock 60.

In der Figur 6 ist eine Variante gezeigt, bei der an einem Basisfunktionsblock 40 mehrere Schnittstellen 46 vorgesehen sind, an die mehrere Zusatzfunktionsblöcke 60 ankoppeln können. Dabei ist es möglich, dass ein Zusatzfunktionsblock 60 die Ankopplung weiterer Zusatzfunktionsblöcke verhindert, also eine Ankopplung weiterer Zusatzfunktionsblöcke damit nicht mehr möglich ist. Bei einer anderen Funktionalität eines Zusatzfunktionsblockes 60 ist es möglich, dass mehrere Zusatzfunktionsblöcke nebeneinander alternativ oder in Kombination miteinander an den Schnittstellen 46 angeordnet und damit kombiniert werden können.

Die Figur 7 zeigt eine Variante dieser blockierenden Schnittstellenausgestaltungen. An dem Basisfunktionsblock 40 sind zwei unterschiedliche Schnittstellen 46 angeordnet oder ausgebildet, an denen jeweils nur ein Zusatzfunktionsblock 60 mit der entsprechenden passenden Schnittstelle 64 angekoppelt werden kann.

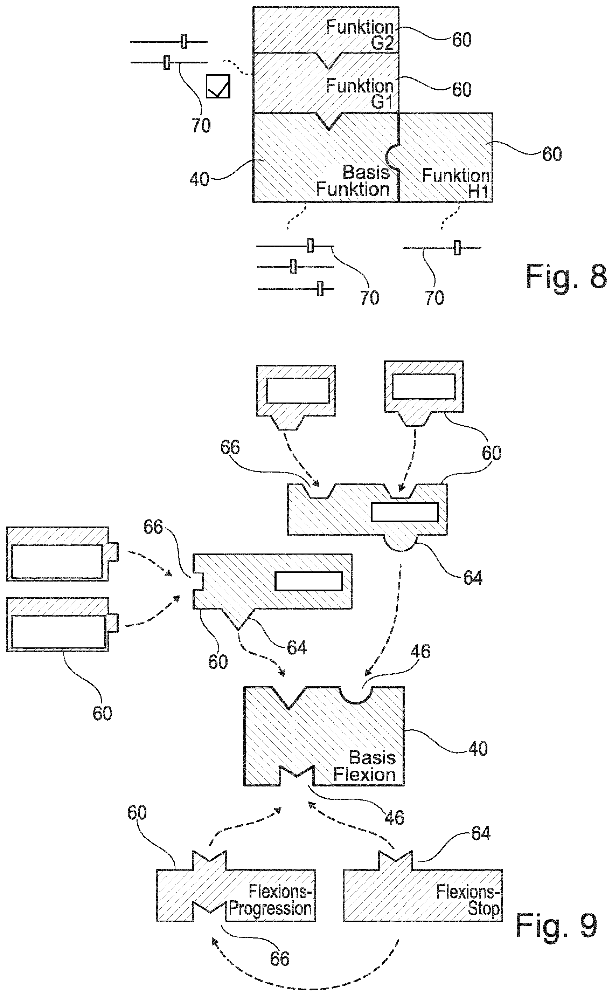

In der Figur 8 ist eine Weiterbildung der Erfindung gezeigt, bei der der Basisfunktionsblock 40 mit insgesamt drei Zusatzfunktionsblöcken 60 gekoppelt ist. Die jeweiligen Funktionalitäten werden auf Kompatibilität überprüft und eine Gesamtfunktionalität wird erzeugt. Zur Anpassung an die unterschiedlichen Bedürfnisse eines Nutzers ist es möglich, über Regler 70 oder Verstelleinrichtungen entsprechende Parameter der jeweiligen Funktionalität einzustellen. Auch diese Parametrisierung wird auf Kompatibilität zu den Einstellungen der anderen Funktionalitäten hin überprüft. Dabei wird ausgeschlossen, dass durch eine irrtümliche Anpassung einer Funktionalität eine ungewollte Wechselwirkung mit einer anderen Funktionalität erzeugt wird, die die Sicherheit der Benutzung der orthopädietechnischen Einrichtung 1 gefährden würde. In der Basisfunktionalität des Basisfunktionsblockes 40 kann auch abgelegt sein, welche Parameter in welchem Parameterbereich verstellt werden können. Wird eine Verstellung über diesen Parameterbereich oder über diesen Wertebereich hinaus durchgeführt, kann dies unterbrochen oder verhindert werden. Über ein Warnsignal kann zudem eine Rückmeldung gegeben werden, dass eine solche Kombination aus Basisfunktionalität und Zusatzfunktionalitäten in einem bestimmten Parameterbereich nicht möglich ist. Wird eine Verstellung akzeptiert, kann eine entsprechende positive Rückmeldung erfolgen.

In der Figur 9 ist ein Ausführungsbeispiel der modularen Bauweise von Basisfunktionalitäten und Zusatzfunktionalitäten dargestellt. Ein Basisfunktionsblock 40 mit drei unterschiedlichen Schnittstellen 46 ist dargestellt. Ist als Basisfunktionalität eine Steuerung eines Prothesenkniegelenkes hinterlegt, kann durch die Hinzufügung eines Zusatzfunktionsblockes 60 z.B. ein Flexionsstop eingestellt oder verstellbar eingestellt werden. Eine Anbindung eines solchen Zusatzfunktionsblockes würde eine Verstellbarkeit oder Einstellbarkeit hinsichtlich der Position des Flexionsstopps unterbinden. Es besteht daher auch die Möglichkeit, eine Flexionsprogression über einen alternativen Zusatzfunktionsblock der Basisfunktionalität hinzuzufügen, an die ein Flexionsstop angekoppelt werden kann, da ein solcher Zusatzfunktionsblock 60 eine weitere Schnittstelle 66 zum Ankoppeln und Kombinieren mit dem Flexionsstoppbaustein aufweist.

Weiterhin können an einer der beiden verbliebenen Schnittstellen 46 andere Steuerungsblöcke oder Funktionselemente angekoppelt werden. Der eine Zusatzfunktionsblock weist nur eine Zusatzschnittstelle 66 auf, an der nur ein weiteres Modul als Erweiterung oder Einschränkung oder zur Modifikation der Zusatzfunktion angekoppelt werden kann. Wird statt des ersten Zusatzfunktionsblockes mit nur einer Zusatzschnittstelle ein anderer Zusatzfunktionsblock 60 mit zwei Zusatzschnittstellen 66 angekoppelt, sind zwei weitere Zusatzfunktionsblöcke 60 alternativ oder in Kombination ankoppelbar.

Bei mehreren Modi, z.B. Gehen oder Radfahren bei orthopädietechnischen Einrichtungen der unteren Extremität oder bei unterschiedlichen Griffarten bei Prothesenhänden, kann jeder Modus aus einer Basis- und Zusatzfunktion zusammengesetzt werden. Es ist aber auch möglich, dass nicht jeder Modus frei programmierbar ist, sondern nur einzelne Modi. Das Umschalten zwischen den Modi erfolgt beispielsweise über Biosignale wie Kokontraktionen, Kontraktionsmuster, Bewegungsmuster, über ein Interface, das z.B. über App angesteuert werden kann oder auf eine andere, ggf. ebenfalls frei programmierte Art und Weise.

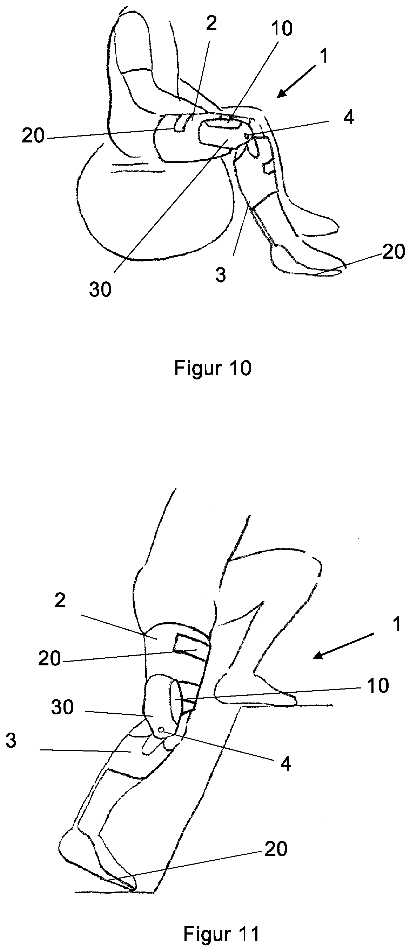

In den Figuren 10 und 11 ist eine orthopädietechnische Einrichtung 1 in Gestalt einer Orthese dargestellt. Der Nutzer der orthopädietechnischen Einrichtung 1 sitzt in der Figur 10, in der Figur 11 ist das Überwinden eines Hindernisses dargestellt. Die Orthese weist ein Oberteil 2 und ein Unterteil 3 auf, die um eine Schwenkachse 4 gelenkig miteinander verbunden sind. Das Oberteil 2 ist als Oberschenkelmanschette ausgebildet, das Unterteil 3 als eine Aufnahmeschale für den Unterschenkel und den Fuß. Über geeignete Befestigungselemente, beispielsweise Riemen, Gurte, Klettverschlüsse, Schnallen oder Schnappverschlüsse wird die orthopädietechnische Einrichtung 1 an dem Oberschenkel und dem Unterschenkel des Orthesennutzers angelegt. Sowohl an dem Oberteil 2 als auch an dem Unterteil 3 sind Sensoren 20 angeordnet, die nur schematisch dargestellt sind. Die Sensoren 20 können beispielsweise als Raumlagesensoren, Drucksensoren, Beschleunigungssensoren oder andere Sensoren ausgebildet sein, um Belastungsdaten, Geschwindigkeitsdaten und andere physikalische Größen aufzunehmen. Ebenfalls ist es möglich, dass die Sensoren 20 Temperaturen aufnehmen. Die Sensoren 20 sind entweder per Kabel oder drahtlos mit einer Datenverarbeitungseinrichtung 10 gekoppelt, die im dargestellten Ausführungsbespiel an dem Oberteil 2 befestigt oder ausgebildet ist. Die Datenverarbeitungseinrichtung 10 verarbeitet die Sensordaten und ist mit den notwendigen Softwarekomponenten und Hardwarekomponenten ausgestattet. Die Datenverarbeitungseinrichtung 10 ist mit einem schematisch dargestellten Aktuator 30 gekoppelt, über den es möglich ist, Verstellungen an Widerstandseinrichtungen wie Dämpfern, magnetorheologische Bremsen oder dergleichen vorzunehmen oder das Oberteil 2 relativ zu dem Unterteil 3 zu verlagern. Dazu ist dann der Aktuator 30 als Antrieb oder Motor ausgebildet. Ebenfalls ist es möglich, dass der Aktuator 30 als ein schaltbarer Energiespeicher ausgebildet ist, beispielsweise eine Feder, die gespannt und entspannt werden kann. Wie oben bei den Ausführungen zu den Figuren 1 und 2 beschrieben, können auch bei einer orthopädietechnischen Einrichtung 1 in Gestalt einer Orthese der unteren Extremität situationsbedingt unterschiedliche Funktionsmodi oder Bewegungsmodi eingestellt werden. Dies kann entweder autonom durch den Orthesennutzer oder in Abhängigkeit von den Sensordaten automatisch erfolgen. Wird beispielsweise durch einen Raumlagesensor 20 an dem Oberteil 2 eine im Wesentlichen waagerechte Orientierung des Oberteils 2 bei gleichzeitiger Entlastung des Unterteils 3 und Abwesenheit einer Relativbewegung um die Schwenkachse 4 erkannt, kann automatisch ein Sitzmodus eingeschaltet werden, der den Aktuator 30 entsprechend aktiviert oder deaktiviert, sodass eine ungedämpfte oder nahezu ungedämpfte freie Beweglichkeit um die Schwenkachse 4 ermöglicht wird. Wird hingegen eine gestreckte Stellung bei einer Vorderfußbelastung wie in der Figur 11 erkannt, kann auf ein Übersteigen eines Hindernisses rückgeschlossen werden, sodass entsprechend andere Widerstände gegen eine Extension oder Flexion oder auch eine Unterstützungskraft durch den Aktuator 30 bereitgestellt werden. Der Aufbau und die Ausgestaltung mit Basisfunktionsblöcken und Zusatzfunktionsblöcken, wie anhand der Figuren 1 und 2 am Beispiel der Prothesen beschrieben wurde, ist auf die Ausführungsform als Orthese entsprechend angewendet. Auch bei Orthesen können Zusatzfunktionsblöcke an entsprechende Schnittstellen angekoppelt werden.

Diese Zusatzfunktionsblöcke können eine weitere Ankopplung von Zusatzfunktionsblöcken an sich selbst ausschließen oder ermöglichen. Es können mehrere Schnittstellen an einem Basisfunktionsblock vorgesehen sein, die alternativ oder ergänzend mit Zusatzfunktionsblöcken verbunden werden können.

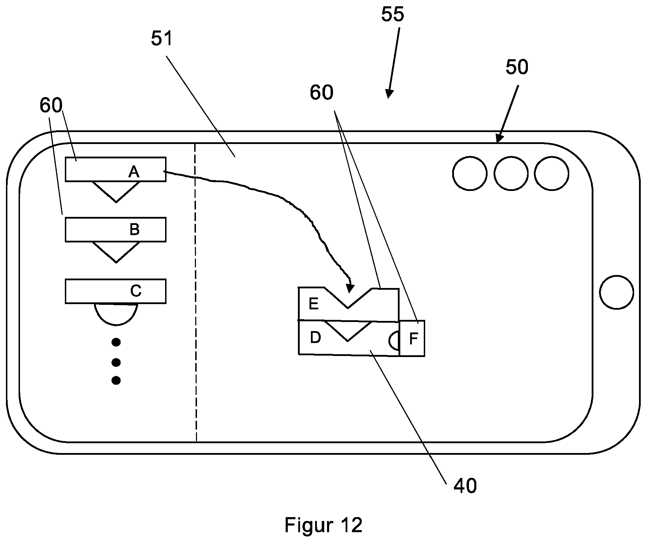

In der Figur 12 ist in einer Detaildarstellung ein externes Gerät 55 in Gestalt eines Mobiltelefones oder eines Tablets dargestellt. Das externe Gerät 55 weist eine Interface-Einrichtung 50 auf, die als ein grafisches Benutzerinterface ausgebildet ist. Ein berührungsempfindliches Display ist an dem externen Gerät 55 ausgebildet und ermöglicht neben einer optischen Informationsübertragung an einen Nutzer eine Eingabe über einen berührungsempfindlichen Bildschirm. Ebenfalls sind an dem externen Gerät 55 eine nicht dargestellte Lautsprechereinrichtung sowie ein Mikrofon vorhanden, um akustische Signale aufzunehmen bzw. über eine Sprachsteuerung eine entsprechende Kombination unterschiedlicher Funktionalitäten vorzunehmen.

Auf dem Display 51 sind verschiedene Zusatzfunktionsblöcke 60 dargestellt, die mit einem Basisfunktionsblock 40 kombinierbar sind. Der Basisfunktionsblock 40 ist in einer zentralen Position auf dem Display 51 dargestellt, zur Verfügung stehende Zusatzfunktionsblöcke 60 sind untereinander in einem abgetrennten Bereich des Displays 51 daneben angeordnet. Die im abgetrennten Bereich darstellten Funktionsblöcke 60 können dabei dynamisch angepasst werden, beispielsweise um nur Funktionsblöcke anzuzeigen die der Konfiguration hinzugefügt werden können. In dem dargestellten Ausführungsbeispiel sind drei Zusatzfunktionsblöcke 60 untereinander angeordnet, die mit unterschiedlichen Funktionalitäten versehen sind und mit dem Buchstaben A bis C gekennzeichnet sind. Durch die darunterstehenden Punkte ist angedeutet, dass beispielsweise durch Scrollen weitere Zusatzfunktionsblöcke 60 in das Display 51 bewegt werden können, um diese dann auszuwählen. Per Drag-and-Drop, was durch den Pfeil angedeutet ist, kann ein Zusatzfunktionsblock A an die Schnittstelle eines weiteren Zusatzfunktionsblockes 60, nämlich des Zusatzfunktionsblockes E, angeschlossen und hinzugefügt werden. Alternativ ist es möglich durch Auswahl einer der Schnittstellen, beispielsweise der oberen Schnittstelle des Funktionsblocks E durch Berührung des Displays, im abgetrennten Bereich eine Auswahl von Funktionsblöcken angezeigt zu bekommen, welche an dieser Schnittstelle hinzugefügt werden können. Durch Drag-and-Drop oder durch einfache Auswahl wird der entsprechende Funktionsblock hinzugefügt. An dem Basisfunktionsblock 40 sind bereits zwei Zusatzfunktionsblöcke 60 angeordnet. Ebenso können bereits vorhandene Zusatzfunktionsblöcke 60 wieder entfernt und somit eine neue Gesamtkonfiguration mit einer neuen Gesamtfunktionalität erzeugt werden. Durch zusätzliche Menüelemente, die durch die Kreise im oberen rechten Eck des Displays 51 angedeutet sind, können erstellte Konfigurationen gespeichert, in die Cloud oder an die orthopädische Einrichtung übertragen, mit Dritten geteilt oder geladen werden

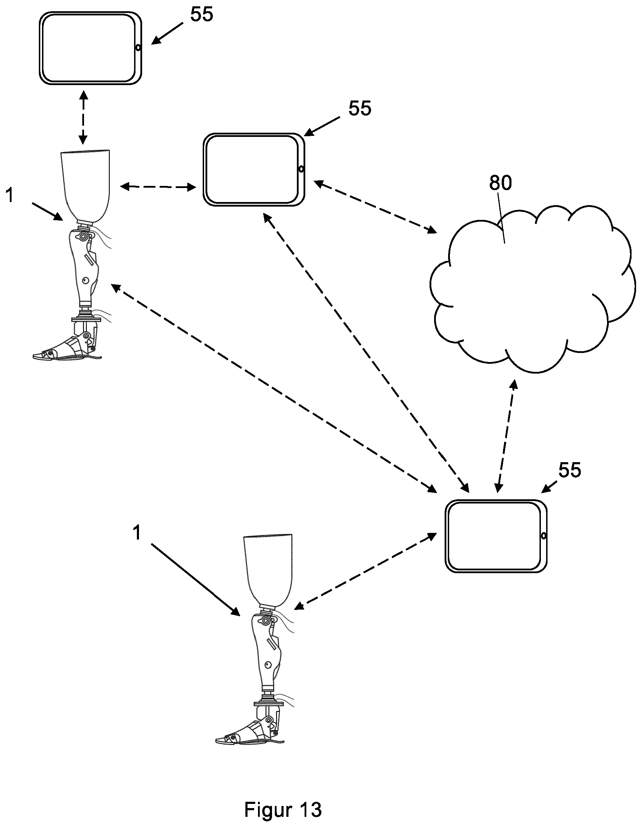

In der Figur 13 ist eine schematische Darstellung einer Vernetzung mehrerer orthopädietechnischer Einrichtungen 1 gezeigt, die miteinander und mit einer externen IT-Infrastruktur 80 verbunden sind. Die IT-Infrastruktur 80 ist vorteilhafterweise als eine sogenannte Cloud ausgebildet und ist ein Rechnernetzwerk, mit dem Speicherplatz, Rechenleistung und/oder Anwendersoftware verfügbar gemacht werden. Ein externes Gerät 55, beispielsweise ein Tablet, ein Mobiltelefon, ein Laptop oder eine andere entsprechende Datenverarbeitungseinrichtung, ist bidirektional mit einer ersten orthopädietechnischen Einrichtung 1, in dem dargestellten Ausführungsbeispiel mit einer Prothese, verbunden. Das erste externe Gerät 55 kann beispielsweise ein Smartphone des Nutzers der orthopädietechnischen Einrichtung sein. Darüber hinaus kann die erste orthopädietechnische Einrichtung 1 mit einem weiteren mobilen Endgerät, einem Tablet oder einem Computer datentechnisch verbunden sein, beispielsweise über eine drahtlose Schnittstelle, eine Funkverbindung oder eine ähnliche Datenübertragungseinrichtung. Von dort werden die Daten in die sogenannte Cloud 80 übermittelt und gegebenenfalls verarbeitet und wieder zurück übertragen. Ebenfalls ist es vorgesehen, dass die Zusatzfunktionsblöcke oder Einstellungen der ersten orthopädietechnischen Einrichtung 1 ausgewertet, an Dritte übertragen und/oder gespeichert werden. Aus der Cloud 80 können die Daten an ein weiteres externes Gerät 55 übermittelt werden, das mit einer zweiten orthopädietechnischen Einrichtung 1 gekoppelt ist. Alternativ zu dem Zwischenschritt oder ergänzend dazu, bei dem die Daten über die Cloud 80 von der ersten orthopädietechnischen Einrichtung 1 auf die zweite orthopädietechnische Einrichtung 1 übertragen werden, kann die Datenübertragung auch direkt über das zweite externe Gerät 55 oder direkt über das dritte externe Gerät 55 auf das erste externe Gerät 55 und zurück erfolgen.

In einem der anderen externen Geräte 55 oder in der Cloud 80 kann eine Überprüfung der Kompatibilität der Funktionsblöcke durchgeführt werden, sodass Rechnerkapazität in der orthopädietechnischen Einrichtung 1 dafür nicht benutzt werden muss. Ebenso können aus den anderen externen Geräten 55 und/oder der Cloud 80 vordefinierte Programme oder Vorlagen an die jeweilige orthopädietechnische Einrichtung 1 oder an ein direkt zugeordnetes externes Gerät 55 oder ein unmittelbar an der orthopädietechnischen Einrichtung 1 angeordnete Interface-Einrichtung übertragen werden. Es findet eine Vernetzung der orthopädietechnischen Einrichtungen und ein Datenaustausch und einer Datenauswertung statt.

## Patentansprüche

1. Verfahren zum Einrichten einer Steuerung einer orthopädietechnischen Einrichtung, die zumindest eine Datenverarbeitungseinrichtung (10), daran gekoppelte Sensoren (20) und einen Aktuator (30) aufweist, der durch die Datenverarbeitungseinrichtung (10) aktiviert und/oder deaktiviert wird, wobei die Steuerung einen Basisfunktionsblock (40) aufweist, in dem eine Basisfunktionalität des Aktuators (30) festgelegt ist, **dadurch gekennzeichnet, dass** aus einem Speicher (15) mehrere Zusatzfunktionsblöcke (60), die unterschiedliche Zusatzfunktionalitäten aufweisen, einer Interfaceeinrichtung (50) bereitgestellt werden und dass über die Interfaceeinrichtung (50) zumindest ein Zusatzfunktionsblock (60) ausgewählt und dem Basisfunktionsblock (40) hinzugefügt wird, wobei eine Schnittstelle (46) zwischen dem Basisfunktionsblock (40) und dem zumindest einen Zusatzfunktionsblock (60) vorhanden ist, an der die Kompatibilität der Funktionalitäten überprüft und eine Gesamtfunktionalität erzeugt wird, wobei bei anfänglich nicht vorhandener Kompatibilität eine Anpassung zumindest einer Funktionalität zur Herstellung der Kompatibilität vorgenommen oder eine Einbindung der Zusatzfunktionalität abgelehnt wird.

2. Verfahren nach Anspruch 1, **dadurch gekennzeichnet, dass** mehrere Zusatzfunktionsblöcke (60) nacheinander dem Basisfunktionsblock (40) hinzugefügt werden und bei jedem neuen Zusatzfunktionsblock eine Überprüfung der Kompatibilität mit der gegenwärtig vorhandenen Gesamtfunktionalität erfolgt.

3. Verfahren nach Anspruch 1 oder 2, **dadurch gekennzeichnet, dass** über die Interfaceeinrichtung (50) zumindest ein Parameter der Basisfunktionalität und/oder Zusatzfunktionalität verstellbar ist.

4. Verfahren nach einem der voranstehenden Ansprüche, **dadurch gekennzeichnet, dass** jeder Zusatzfunktionalität ein Priorisierungswert zugeordnet wird und eine Überprüfung der Kompatibilität und Anpassung der jeweiligen Funktionalität anhand der Priorisierungswerte vorgenommen wird.

5. Verfahren nach einem der voranstehenden Ansprüche, **dadurch gekennzeichnet, dass** als Interfaceeinrichtung (50) eine graphische Benutzeroberfläche (51) bereitgestellt wird, auf der die Zusatzfunktionalitäten (60) als Benutzeroberflächenobjekte (52) dargestellt sind.

6. Verfahren nach Anspruch 5, **dadurch gekennzeichnet, dass** die Kompatibilität verschiedener Zusatzfunktionsblöcke (60) miteinander oder mit der aktuellen Kombination aus Basisfunktionsblock (40) und zumindest einem Zusatzfunktionsblock (60) graphisch angezeigt wird.

7. Verfahren nach Anspruch 5 oder 6, **dadurch gekennzeichnet, dass** an den Benutzeroberflächenobjekten eine farbliche und/oder graphische Kombinierbarkeit eines Zusatzfunktionsblockes mit dem Basisfunktionsblock (40) und/oder einem anderen Zusatzfunktionsblock (60) dargestellt wird.

8. Verfahren nach einem der voranstehenden Ansprüche, **dadurch gekennzeichnet, dass** es auf der Datenverarbeitungseinrichtung (10) implementiert ist.

9. Verfahren nach einem der voranstehenden Ansprüche, **dadurch gekennzeichnet, dass** an einem Zusatzfunktionsblock (60) eine Schnittstelle (66) für zumindest einen weiteren Zusatzfunktionsblock (60) ausgebildet ist.

10. Verfahren nach einem der voranstehenden Ansprüche, **dadurch gekennzeichnet, dass** der Basisfunktionsblock (40) und/oder ein Zusatzfunktionsblock (60) mehrere Schnittstellen (46, 66) aufweist, von denen zumindest eine durch eine Belegung einer anderen Schnittstelle mit einen Zusatzfunktionsblock (60) blockiert wird.

11. Verfahren nach einem der voranstehenden Ansprüche, **dadurch gekennzeichnet, dass** die Überprüfung der Kompatibilität der Funktionsblöcke (40, 60) und/oder die Integration mehrerer Funktionsblöcke (40, 60) in eine Gesamtfunktionalität in einem externen Gerät (55) erfolgt.

12. Verfahren nach einem der voranstehenden Ansprüche, **dadurch gekennzeichnet, dass** vordefinierte Programme oder Vorlagen zur Erstellung eines Funktionsblockes (40, 60) verwendet werden.

13. Verfahren nach einem der voranstehenden Ansprüche, **dadurch gekennzeichnet, dass** die Datenverarbeitungseinrichtung (10) über eine Kommunikationsschnittstelle (11) Daten an externe Einrichtungen (55) sendet und/oder von externen Einrichtungen (55) empfängt.

14. Orthopädietechnische Einrichtung (1) mit einer Datenverarbeitungseinrichtung (10), die zum Einrichten einer Steuerung der orthopädietechnischen Einrichtung (1) eingerichtet und ausgebildet ist, an der Datenverarbeitungseinrichtung (10) sind Sensoren (20) gekoppelt und die orthopädietechnische Einrichtung (1) weist einen Aktuator (30) auf, der mit der Datenverarbeitungseinrichtung (10) gekoppelt ist und durch die Datenverarbeitungseinrichtung (10) aktiviert und/oder deaktiviert wird, wobei die Steuerung einen Basisfunktionsblock (40) aufweist, in dem eine Basisfunktionalität des Aktuators (30) festgelegt ist, wobei die orthopädietechnische Einrichtung (1) eine Kommunikationsschnittstelle aufweist, über die aus einem Speicher (15) an der orthopädietechnischen Einrichtung (1) mehrere Zusatzfunktionsblöcke (60), die unterschiedliche Zusatzfunktionalitäten aufweisen, einer mit der orthopädietechnischen Einrichtung gekoppelten Interfaceeinrichtung (50) bereitgestellt werden und über die Interfaceeinrichtung (50) zumindest ein Zusatzfunktionsblock (60) ausgewählbar und dem Basisfunktionsblock (40) hinzufügbar ist, wobei eine Schnittstelle (46) zwischen dem Basisfunktionsblock (40) und dem zumindest einen Zusatzfunktionsblock (60) vorhanden ist, an der die Kompatibilität der Funktionalitäten überprüfbar und eine Gesamtfunktionalität erzeugbar ist, wobei bei anfänglich nicht vorhandener Kompatibilität eine Anpassung zumindest einer Funktionalität zur Herstellung der Kompatibilität vorgenommen oder eine Einbindung der Zusatzfunktionalität abgelehnt wird, mit einer Benutzeroberfläche (51), mit Eingabemitteln zur Entgegennahme von Benutzereingaben.

15. Computerprogrammprodukt mit einem Programmcode, der, wenn er in einer Datenverarbeitungseinrichtung (10) einer Steuerung einer orthopädietechnischen Einrichtung geladen ist, wobei die Datenverarbeitungseinrichtung (10) die mit Sensoren (20) gekoppelt ist und einen Aktuator (30) aktiviert und/oder deaktiviert und die Steuerung einen Basisfunktionsblock (40) aufweist, in dem eine Basisfunktionalität des Aktuators (30) festgelegt ist, zur Ausführung bringt, dass aus einem Speicher (15) mehrere Zusatzfunktionsblöcke (60), die unterschiedliche Zusatzfunktionalitäten aufweisen, einer Interfaceeinrichtung (50) bereitgestellt werden und dass über die Interfaceeinrichtung (50) zumindest ein Zusatzfunktionsblock (60) ausgewählt und dem Basisfunktionsblock (40) hinzugefügt wird, wobei eine Schnittstelle (46) zwischen dem Basisfunktionsblock (40) und dem zumindest einen Zusatzfunktionsblock (60) vorhanden ist, an der die Kompatibilität der Funktionalitäten überprüft und eine Gesamtfunktionalität erzeugt wird, wobei bei anfänglich nicht vorhandener Kompatibilität eine Anpassung zumindest einer Funktionalität zur Herstellung der Kompatibilität vorgenommen oder eine Einbindung der Zusatzfunktionalität abgelehnt wird.

## Claims

1. A method for configuring a control for an orthopedic device having at least one data processing device (10), sensors (20) coupled thereto, and an actuator (30) which is activated and/or deactivated by the data processing device (10), the control having a basic function block (40) in which a basic functionality of the actuator (30) is defined, **characterized in that** a plurality of add-on function blocks (60) which have different add-on functionalities are made available to an interface device (50) from a memory (15) and **in that** at least one add-on function block (60) is selected via the interface device (50) and added to the basic function block (40), there being an interface (46) between the basic function block (40) and the at least one add-on function block (60), at which the compatibility of the functionalities is checked and an overall functionality is generated, with, in the case of a lack of compatibility at the outset, at least one functionality being adapted to produce the compatibility or integration of the add-on functionality being rejected.

2. The method as claimed in claim 1, **characterized in that** a plurality of add-on function blocks (60) are successively added to the basic function block (40) and the compatibility with the currently existing overall functionality is checked for each new add-on function block.

3. The method as claimed in claim 1 or 2, **characterized in that** at least one parameter of the basic functionality and/or add-on functionality is adjustable via the interface device (50).

4. The method as claimed in any one of the preceding claims, **characterized in that** each add-on functionality is assigned a prioritization value and the compatibility and adaptation of the respective functionality is checked using the prioritization values.

5. The method as claimed in any one of the preceding claims, **characterized in that** a graphical user interface (51) is provided as the interface device (50), the add-on functionalities (60) being displayed on said graphical user interface as user interface objects (52).

6. The method as claimed in claim 5, **characterized in that** the compatibility of different add-on function blocks (60) with one another or with the current combination of basic function block (40) and at least one add-on function block (60) is displayed graphically.

7. The method as claimed in claim 5 or 6, **characterized in that** a color and/or graphic combinability of an add-on function block with the basic function block (40) and/or another add-on function block (60) is displayed on the user interface objects.

8. The method as claimed in any one of the preceding claims, **characterized in that** the said method is implemented on the data processing device (10).

9. The method as claimed in any one of the preceding claims, **characterized in that** an interface (66) for at least one further add-on function block (60) is formed on an add-on function block (60).

10. The method as claimed in any one of the preceding claims, **characterized in that** the basic function block (40) and/or an add-on function block (60) has a plurality of interfaces (46, 66), at least one of which is blocked as a result of another interface being occupied with an add-on function block (60).

11. The method as claimed in any one of the preceding claims, **characterized in that** the compatibility of the function blocks (40, 60) and/or the integration of a plurality of function blocks (40, 60) into an overall functionality is checked in an external apparatus (55).

12. The method as claimed in any one of the preceding claims, **characterized in that** predefined programs or templates are used to create a function block (40, 60).

13. The method as claimed in any one of the preceding claims, **characterized in that** the data processing device (10) transmits data to external devices (55) and/or receives data from external devices (55) via a communication interface (11).

14. An orthopedic device (1) comprising a data processing device (10) which is configured and designed for configuring a control for an orthopedic device (1) having at least one data processing device (10), sensors (20) coupled thereto, and the orthopedic device (1) comprises an actuator (30) which coupled to the data processing device (10) is activated and/or deactivated by the data processing device (10), the control having a basic function block (40) in which a basic functionality of the actuator (30) is defined, wherein the orthopedic device (1) comprises a communication interface by which a plurality of add-on function blocks (60) which have different add-on functionalities are made available to an interface device (50) of the orthopedic device (1) from a memory (15) of the orthopedic device (1) and in that at least one add-on function block (60) is selectable via the interface device (50) and is addable to the basic function block (40), there being an interface (46) between the basic function block (40) and the at least one add-on function block (60), at which the compatibility of the functionalities is checkable and an overall functionality is generatable, with, in the case of a lack of compatibility at the outset, at least one functionality being adapted to produce the compatibility or integration of the add-on functionality being rejected, and comprising a user interface (51) with input means for receiving user inputs.

15. A computer program product comprising a program code which causes, when loaded into a data processing device (10) of a control for an orthopedic device, wherein the data processing device (10) is coupled to sensors (20) and activates and/or deactivates an actuator (30), the control having a basic function block (40) in which a basic functionality of the actuator (30) is defined, that a plurality of add-on function blocks (60) which have different add-on functionalities are made available to an interface device (50) from a memory (15) and in that at least one add-on function block (60) is selected via the interface device (50) and added to the basic function block (40), there being an interface (46) between the basic function block (40) and the at least one add-on function block (60), at which the compatibility of the functionalities is checked and an overall functionality is generated, with, in the case of a lack of compatibility at the outset, at least one functionality being adapted to produce the compatibility or integration of the add-on functionality being rejected.

## Revendications

1. Procédé pour configurer une unité de commande d'un dispositif orthopédique, comprenant au moins une unité de traitement de données (10), des capteurs (20) couplés à celle-ci, et un actionneur (30) qui est activé et/ou désactivé par l'unité de traitement de données (10), l'unité de commande comportant un bloc fonctionnel de base (40) dans lequel est définie une fonctionnalité de base de l'actionneur (30),
**caractérisé en ce que** plusieurs blocs fonctionnels supplémentaires (60) enregistrés dans une mémoire (15) et présentant différentes fonctionnalités supplémentaires sont fournis à un dispositif d'interface (50), et **en ce qu'**au moins un bloc fonctionnel supplémentaire (60) est sélectionné via le dispositif d'interface (50) et est ajouté au bloc fonctionnel de base (40), sachant qu'il est prévu une interface (46) entre le bloc fonctionnel de base (40) et ledit au moins un bloc fonctionnel supplémentaire (60), au niveau de laquelle la compatibilité des fonctionnalités est vérifiée et une fonctionnalité globale est créée, et en cas d'incompatibilité initiale, une adaptation d'au moins une fonctionnalité est effectuée pour établir la compatibilité ou bien l'intégration de la fonctionnalité supplémentaire est refusée.

2. Procédé selon la revendication 1,
**caractérisé en ce que** plusieurs blocs fonctionnels supplémentaires (60) sont ajoutés successivement au bloc fonctionnel de base (40), et pour chaque nouveau bloc fonctionnel supplémentaire, une vérification de la compatibilité avec la fonctionnalité globale actuellement disponible est effectuée.

3. Procédé selon la revendication 1 ou 2,
**caractérisé en ce qu'**au moins un paramètre de la fonctionnalité de base et/ou de la fonctionnalité supplémentaire peut être réglé via le dispositif d'interface (50).

4. Procédé selon l'une des revendications précédentes,
**caractérisé en ce qu'**une valeur de priorité est attribuée à chaque fonctionnalité supplémentaire, la vérification de la compatibilité et l'adaptation de la fonctionnalité respective étant effectuées à l'aide des valeurs de priorité.

5. Procédé selon l'une des revendications précédentes,
**caractérisé en ce qu'**une interface utilisateur graphique (51) est fournie comme dispositif d'interface (50), sur laquelle les fonctionnalités supplémentaires (60) sont représentées comme des objets d'interface utilisateur (52).

6. Procédé selon la revendication 5,
**caractérisé en ce que** la compatibilité de différents blocs fonctionnels supplémentaires (60) entre eux ou avec la combinaison actuelle d'un bloc fonctionnel de base (40) et d'au moins un bloc fonctionnel supplémentaire (60) est affichée graphiquement.

7. Procédé selon la revendication 5 ou 6,
**caractérisé en ce que** la possibilité de combinaison d'un bloc fonctionnel supplémentaire avec le bloc fonctionnel de base (40) et/ou avec un autre bloc fonctionnel supplémentaire (60) est représentée par voie chromatique et/ou graphique sur les objets d'interface utilisateur.

8. Procédé selon l'une des revendications précédentes,
**caractérisé en ce qu'**il est mis en œuvre sur l'unité de traitement de données (10).

9. Procédé selon l'une des revendications précédentes,
**caractérisé en ce qu'**une interface (66) pour au moins un autre bloc fonctionnel supplémentaire (60) est formée sur un bloc fonctionnel supplémentaire (60).

10. Procédé selon l'une des revendications précédentes,
**caractérisé en ce que** le bloc fonctionnel de base (40) et/ou un bloc fonctionnel supplémentaire (60) comporte plusieurs interfaces (46, 66), dont au moins une est bloquée par l'affectation d'un bloc fonctionnel supplémentaire (60) à une autre interface.

11. Procédé selon l'une des revendications précédentes,
**caractérisé en ce que** la vérification de la compatibilité des blocs fonctionnels (40, 60) et/ou l'intégration de plusieurs blocs fonctionnels (40, 60) dans une fonctionnalité globale s'effectue dans un appareil externe (55).

12. Procédé selon l'une des revendications précédentes,
**caractérisé en ce que** des programmes ou des modèles prédéfinis sont utilisés pour créer un bloc fonctionnel (40, 60).

13. Procédé selon l'une des revendications précédentes,
**caractérisé en ce que** l'unité de traitement de données (10) envoie des données à des dispositifs externes (55) via une interface de communication (11) et/ou les reçoit en provenance de dispositifs externes (55).

14. Dispositif orthopédique (1) comprenant une unité de traitement de données (10) conçue et agencée pour configurer une unité de commande d'un dispositif orthopédique,
dans lequel
des capteurs (20) sont couplés à l'unité de traitement de données (10), et le dispositif orthopédique (10) comprend un actionneur (30) qui est couplé à l'unité de traitement de données (10) et est activé et/ou désactivé par l'unité de traitement de données (10), l'unité de commande comportant un bloc fonctionnel de base (40) dans lequel est définie une fonctionnalité de base de l'actionneur (30),
le dispositif orthopédique (1) comprend une interface de communication via laquelle plusieurs blocs fonctionnels supplémentaires (60) enregistrés dans une mémoire (15), située au niveau du dispositif orthopédique (1), et présentant différentes fonctionnalités supplémentaires sont fournis à un dispositif d'interface (50) couplée au dispositif orthopédique, et au moins un bloc fonctionnel supplémentaire (60) peut être sélectionné via le dispositif d'interface (50) et être ajouté au bloc fonctionnel de base (40),
il est prévu une interface (46) entre le bloc fonctionnel de base (40) et ledit au moins un bloc fonctionnel supplémentaire (60), au niveau de laquelle la compatibilité des fonctionnalités peut être vérifiée et une fonctionnalité globale peut être créée, et en cas d'incompatibilité initiale, une adaptation d'au moins une fonctionnalité est effectuée pour établir la compatibilité ou bien l'intégration de la fonctionnalité supplémentaire est refusée,
comprenant une interface utilisateur (51) présentant des moyens d'entrée pour recevoir des entrées utilisateur.

15. Produit de programme informatique avec un code de programme qui,
lorsqu'il est chargé dans une unité de traitement de données (10) d'une unité de commande d'un dispositif orthopédique et lorsque l'unité de traitement de données (10) est couplée aux capteurs (20) et vient activer et/ou désactiver un actionneur (30) et que l'unité de commande comprend un bloc fonctionnel de base (40) dans lequel est définie une fonctionnalité de base de l'actionneur,
met en œuvre que plusieurs blocs fonctionnels supplémentaires (60) enregistrés dans une mémoire (15) et présentant différentes fonctionnalités supplémentaires sont fournis à un dispositif d'interface (50), et qu'au moins un bloc fonctionnel supplémentaire (60) est sélectionné via le dispositif d'interface (50) et est ajouté au bloc fonctionnel de base (40), sachant qu'il est prévu une interface (46) entre le bloc fonctionnel de base (40) et ledit au moins un bloc fonctionnel supplémentaire (60), au niveau de laquelle la compatibilité des fonctionnalités est vérifiée et une fonctionnalité globale est créée, et, en cas d'incompatibilité initiale, une adaptation d'au moins une fonctionnalité est effectuée pour établir la compatibilité ou bien l'intégration de la fonctionnalité supplémentaire est refusée.
